# EUROPEAN PATENT SPECIFICATION

(11) **EP 4 114 977 B1**
(45) Date of publication and mention of the grant of the patent: **11.09.2024**
(21) Application number: 21723451.7
(22) Date of filing: 01.03.2021
(51) Int. Cl.: C12Q 1/6883

(54) **NON-INVASIVE SUCCESSFULNESS TEST OF IN VITRO FERTILIZATION PROCESS**
NICHTINVASIVER TEST ZUM ERFOLG EINES IN-VITRO-BEFRUCHTUNGSVERFAHRENS
TEST NON INVASIF DE RÉUSSITE DU PROCESSUS DE FÉCONDATION IN VITRO

(30) Priority: 02.03.2020 SK 500102020
(43) Date of publication of application: 11.01.2023
(73) Proprietor: Fetus, IVF a.s., 120 00 Praha 2 (CZ)
(72) Inventor: RABAJDOVÁ, Miroslava, 040 01 Kosice 1 (SK); SLABÝ, Ondrej, 602 00 Brno (CZ); SOLTYS, Katarína, 841 02 Bratislava (SK); MAREKOVÁ, Mária, 040 13 Kosice 13 (SK); TOPORCEROVÁ, Silvia, 040 11 Kosice (SK)
(74) Representative: Majlingová, Zuzana
(86) International application number: PCT/SK2021/050004
(87) International publication number: WO 2021/177904

(56) References cited:
- WO-A1-2014/062442
- CN-A- 109 504 784
- US-A1- 2014 296 099
- YANG QIAN ET AL: "Association of the peripheral blood levels of circulating microRNAs with both recurrent miscarriage and the outcomes of embryo transfer in an in vitro fertilization process", JOURNAL OF TRANSLATIONAL MEDICINE, vol. 16, no. 1, 4 July 2018 (2018-07-04), XP055816559, Retrieved from the Internet <URL:http://link.springer.com/content/pdf/10.1186/s12967-018-1556-x.pdf> DOI: 10.1186/s12967-018-1556-x
- RANGELOV I I ET AL: "Micrornas in blastocyst culture medium as noninvasive biomarkers for assessing the implantation potential of human embryos", FERTILITY AND STERILITY, vol. 108, no. 3, 1 September 2017 (2017-09-01), XP085158495, ISSN: 0015-0282, DOI: 10.1016/J.FERTNSTERT.2017.07.259
- "Abstracts for 2017 Foundation for Reproductive Medicine Translational Reproductive Biology and Clinical Reproductive Endocrinology Conference", JOURNAL OF ASSISTED REPRODUCTION AND GENETICS, PLENUM PUBLISHING, US, vol. 34, no. 10, 3 October 2017 (2017-10-03), pages 1385 - 1402, XP036334854, ISSN: 1058-0468, [retrieved on 20171003], DOI: 10.1007/S10815-017-1055-7

## Description

### Technical Field

The present invention relates to the identification of the set of particular miRNA molecules for the determination of IVF (*in vitro* fertilization) process successfulness/failure, their usage for the creation of a diagnostic test for prediction of *in vitro* fertilization process successfulness in women during artificial fertilization.

### Background Art

Human population health is one of the main attributes of functioning of the human society. High social demands result in many negative effects to overall human health. Presently, 48.5 million couples suffer from infertility worldwide. Considering infertility as a problem of the general population, the number of realized assisted reproduction cycles was significantly increased in the last decades in clinical conditions. In spite of a number of technical improvements in *in vitro* embryo cultivation, and in spite of all cultivation media improvements, most of the transferred embryos are not able to nestle in the uterus (de Mouzon et al., 2012). Therefore, the relatively low successfulness of the assisted reproduction methods is improved by the transfer of a higher number of embryos within one IVF cycle. Optimal selection of an embryo in the course of IVF is a critical step of the whole treatment. Further to other factors, particularly a high-quality embryo suitable for IVF process contributes to successful implantation. The embryo quality, except for its basic morphological features (monitored by an embryoscope), is not monitored and diagnosed for the determination of IVF process successfulness. Various selection and assessment systems presently used for assessment of human embryos quality have probably achieved their maximum, and they are not able to improve assisted reproduction results. The percentage of IVF process successfulness is about 29 %. Therefore, it is necessary to search for another marker. An oocyte itself can predict development of an early embryo in a significant way, because it provides RNA and cellular mechanisms. Many scientific groups study follicular fluid and its proteomics in order to predict the embryos' quality (Kosteria et al., 2017). Collection of the follicular fluid from the individual follicles is, however, technically demanding and a complex procedure in the clinical praxis. Furthermore, the probability of fertilization of the collected oocytes does not reach the required percentage, and therefore more analysis and embryonic methods multiply the economic costs at this level.

Further to the analysis of angiogenesis and vascularization markers, the scientific community focuses on the study of non-coding RNA molecules, i. e. miRNA, which regulate post-transcription gene expression in eukaryotic cells. It was shown that more than 130 miRNA are expressed in the human blastocyst and their expression varies depending on ploidy and gender of the embryos. During embryogenesis the specific miRNA molecules are remarkably expressed. Their levels are detectable in the embryo's cultivation material and also during its *in vitro* cultivation. The cultivation media become a "waste material" after the completion of the embryo transfer or after freezing of the embryo. Until now several studies dealing with the relationship between non-perceptive and also receptive endometrium in correlation with embryo development competency in the cultivation medium and miRNA (e. g. miR142-5p, miR146a-5p, miR21, miR19b, miR302c) were published. Nevertheless, no statistically significant and relevant results have been published until now. Discrepancies can be based also on usage of different miRNA analytical methods. Presence/absence of miRNA molecules and their mutual correlation relationship in biologic materials from women in IVF process, and women experiencing repeated implantation failures were not studied until now. It is therefore very needed to create an algorithm for the combination of more miRNA molecules biomarkers, or other non-coding ncRNA, which could eventually determine IVF process successfulness/failure in women. Usage of this information will enable to specify endometrial perceptiveness status, emerging implantation window period, as well as the most suitable time for the embryo transfer in IVF process. Human embryo implementation is a complex and multi-factorial process. miRNAs are endogenous, evolutionary conservative, single stranded non-coding RNA molecules comprising 20-24 nucleotides, which regulate post-transcription gene expression in eukaryotic cells, including mammal cells (Asirvatham et al., 2009; McCallie et al., 2010; Mouillet et al., 2015; Catalanotto et al.,2016). Presently more than 250 human miRNAs are recorded in the miRBase biologic database. The human blastocyst expresses more than 130 miRNAs (McCallie et al., 2010; Rosenbluth et al., 2013, Chan et al., 2018). It was also confirmed that miRNA expression in the human blastocyst varies depending on embryo poidy, as well as on embryo gender (Rosenbluth et al., 2013). miRNA bioinformatics analysis suggests their potential role in the regulation of some biological paths and cell functions, including cell cycle inhibition and tumor suppressing activities (Lee et al., 2014). Biological functions of most miRNAs, however, are not exactly known until now. Several studies investigating the relationship between embryo development competency and miRNAs have been published until now. However, no consensus was established to this day. This discrepancy can be caused by various miRNA analysis methods, times of cultivation medium collection depending on the embryo development day, and a number of other factors. Furthermore, continual synthesis and degradation of miRNA are performed during the division of the early embryo. It was shown that miRNAs are inherited from a mother and up to 60 % of miRNAs are lost during the first zygote division (Tang et al., 2007). mRNA expression increases again at blastocyst stage (Zhang et al., 2016). Due to the complexity and the high number of independent processes running during division of the early embryo, it is not probable that one biomarker could predict the embryo development competency in the course of the *in vitro* fertilization program. It is therefore important to perform studies focusing on the determination of the basic biomarkers combination for the developing embryo before its implantation to uterus, whose combination would correlate with the pregnancy result.

US2014296099 discloses methods for detecting expression of miRNAs in the culture medium of in vitro fertilized embryos in order to assess the likelihood of viability of the embryo, and further select an embryo for subsequent implantation in a patient. Yang Qian et al (J. Transl.Med., 16(1), 2018) disclosed identification of miRNAs as biomarkers for recurrent miscarriage and/or implantation failure to predict outcome of IVF with embryo transfer.

Perceptive endometrium capable of the interaction with the embryo, as well as the determination of the implantation window represent other factors of the multi-factorial IVF process. Presently the non-invasive determination of the endometrium perceptiveness uses particularly ultrasound markers - thickness, volume, and blood flow in the endometrium (Bonilla-Musoles et al., 2013). The implementation window is a time-limited phenomenon in the time-limited secretion phase of menstrual cycle, which is also known as "window of implantation" (IW). Most frequently the implementation window occurs in a short period - between day 20 and day 24 of the ovarian cycle, and it usually lasts only two days. Specific regulation of the expression of selected genes participating in angiogenesis activation, vascularization, and immunity reaction occurs within said period. Present studies directed to the incorrect perceptiveness of the endometrium concluded that different biomarkers' expression, whether increased, or decreased, often results in the perceptiveness disorders. The implantation window is shifted or, in the worst case, it is eliminated. Due to problematic interpretation and predictive value of the presently available tests, currently the endometrium perceptiveness (ER) tests are used only rarely in infertile women or in women before the planed artificial fertilization (IVF). Implementation failure due to the non-perceptive endometrium remains an unsolved issue of reproduction medicine and a very common reason of infertility in otherwise healthy women. During the last 5 years the percentage rate of the successfully implanted embryos is 29 %, in spite of continually increasing efforts to improve the implantation process under *in vitro* conditions. The selection of the proper embryo, determination of the endometrium perceptiveness, and suitable implantation time in the female cycle are the key variables in the *in vitro* fertilization process. There are patented tests for endometrium perceptiveness, as well as for the implantation window. Until now, however, no distribution of the particular canonical miRNA and iso-miRNA molecules analyzed by NGS (Next Generation Sequencing) in the determination of the mother's biological competence at multi-factorial consideration of its variation, or IVF process implantation successfulness prediction in the combination with the selection of the high-quality embryo in the specific day of IVF process was patented.

There is no diagnostic test on the world market that enables the prediction of the successfulness of the subsequent IVF process and prognostic selection of the high-quality embryo suitable for the IVF transfer, within 24 hours on the basis of the assessment of the presence of the canonical and iso-miRNA molecules combination in the woman's plasma and in the spent blastocyst medium. The aim of the invention is to increase the successfulness of the IVF process in artificial fertilization, and to provide a diagnostic test for the prediction of IVF process successfulness.

### Summary of the Invention

The present description relates to the identification of the set of the particular miRNA molecules, i.e. the creation of an miRNA profile for the determination of successfulness/failure of the IVF process. Furthermore, the present disclosure relates to the possible of using the miRNA profile for the production of an innovative diagnostic non-invasive test as a personalized medicine product for *in vitro* fertilization process successfulness prediction in women undergoing artificial fertilization. The diagnostic test includes two independent tests for the identification of prognostic canonic miRNA and iso-miRNA biomarkers. The first one is a Success Test establishing a miRNA profile from peripheral blood plasma of a woman. The second one is a CultMed Test establishing miRNA profile from an embryo's (day 4 to day 6 of its development) cultivation medium. In a standard way the cultivation medium miRNA profile is determined on day 5 of the embryo's development. The spent blastocyst medium can be called as "cultivation waste" or "waste material".

The present invention relates to the particular miRNA molecules identified in a woman's plasma, and in a spent blastocyst medium. The specific canonical miRNA and iso-miRNA molecules exhibited significantly different representation and distribution in women's plasma in the group of a successful IVF process in comparison to the women in the group of an unsuccessful IVF process on the particular day. The distribution of the particular canonical miRNA and iso-miRNA molecules (miRNA profile) in plasma can be used for diagnosis, for the assessment of biological competence - readiness or non-readiness of a mother for the IVF process, on the day of the IVF process. Secretion of the particular canonical and iso-miRNA molecules (miRNA profile) to the spent blastocyst medium can be used for diagnosis on the day of IVF process, and for assessment of the embryo's competence, quality.

Accuracy of IVF process prediction was verified by four models as follows:
Logistic regression - a model using a logistic function (*Sigmoid*) and L2 regularization for binary classification on the basis of the predictive miRNA molecules;
Random Forest - a model using the properties of multiplicatively applied decision trees for binary classification on the basis of the predictive miRNA molecules;
Support Vector Machine (SVM) - a complex model using non-linear method *rbf* together with other parameters for binary classification on the basis of the predictive miRNA molecules;
Stochastic gradient descent (SGD) - a model using an iterative randomly initialized logistic regression method together with SGD learning for binary classification on the basis of the predictive miRNA molecules.

The subject matter of the invention resides in the non-invasive successfulness test of the IVF (*in vitro* fertilization) process consisting of two independent steps:
- step for identification and analysis of the prognostic canonical and iso-miRNA biomarkers from the peripheral blood plasma of women on the day of the IVF process (Success Test), and
- step for prognostic canonical miRNA and iso-miRNA biomarkers from the spent blastocyst medium collected once on day 4 to 6 of the embryo cultivation (CultMed Test),

wherein the successful Success Test shows the cut off values, for the selected miRNA biomarkers at reads number of 1, that are ≥ 1, and the cut off value, for miRNA iso-hsa-let-7b-5p_TGAGGTAGTATGTTGTGTGG at reads number of 5, that is ≥ 5, and the successful CultMed Test shows the cut off value of the selected miRNA biomarkers at reads number of 1, being ≥ 1,
and wherein these values of both tests predicate the successful IVF process. The CultMed test having 95% accuracy with the AUC method includes: taking the spent blastocyst medium sample; isolating all miRNA, followed by performing sequence analysis, and data analysis for the 14 miRNA: iso-hsa-let-7b-5p, iso-hsa-miR-423-5p, iso-hsa- miR-21-5p, iso-hsa-miR-486-5p, iso-hsa-miR-486-5p, iso-hsa-miR-25-3p, iso- hsa-miR-3613-5p, iso-hsa-miR-142-3p, iso-hsa-miR-664a-5p, iso-hsa-miR- 151a-3p, iso-hsa-miR-92a-3p, iso-hsa-let-7a-5p, iso-hsa-let-7b-5p, iso-hsa- miR-100-5p; and determining the reads number for the 14 miRNA biomarkers from the previous step.

One analysis of the non-invasive IVF successfulness test includes the identification of the prognostic canonical miRNA and iso-miRNA biomarkers - Success Test with 100% sensitivity and 100% specificity having average accuracy of 4 different models of 88 %, performed in the following steps:
- Taking a peripheral blood plasma sample that has been obtained from a woman.
- Isolating all miRNA followed by performing sequence analysis, and data analysis for selected 19 miRNA: iso-hsa-miR-126-3p with SEQ ID NO: 1, iso-hsa-let-7i-5p with SEQ ID NO: 2, iso-hsa-miR-342-3p with SEQ ID NO: 3, iso-hsa-let-7b-5p with SEQ ID NO: 4, iso-hsa-miR-132-3p with SEQ ID NO: 5, iso-hsa-miR-23b-3p with SEQ ID NO: 6, iso-hsa-miR-125a-5p with SEQ ID NO: 7, iso-hsa-miR-584-5p with SEQ ID NO: 8, iso-hsa-miR-127-3p with SEQ ID NO: 9, iso-hsa-miR-101-3p with SEQ ID NO: 10, iso-hsa-miR-151a-3p with SEQ ID NO: 11, iso-hsa-miR-92a-3p with SEQ ID NO: 12, iso-hsa-let-7b-5p with SEQ ID NO: 13, iso-hsa-miR-589-5p with SEQ ID NO: 14, iso-hsa-miR-652-3p with SEQ ID NO: 15, iso-hsa-miR-1307-3p with SEQ ID NO: 16, iso-hsa-miR-382-5p with SEQ ID NO: 17, iso-hsa-let-7b-5p with SEQ ID NO: 18, iso-hsa-let-7i-5p with SEQ ID NO: 19.
- Determining the reads number for 19 miRNA biomarkers from the previous step.

In a preferred embodiment, the second part of non-invasive IVF successfulness test analysis includes the identification of the prognostic canonical and iso-miRNA biomarkers - CultMed Test with 100 % sensitivity and 80 % specificity having average accuracy of 4 different models of 85 %, performed in the following steps:
- Taking a spent blastocyst medium sample.
- Isolating all miRNA followed by performing sequence analysis, and data analysis for selected 17 miRNA: iso-hsa-let-7b-5p with SEQ ID NO: 20, iso-hsa-miR-423-5p with SEQ ID NO: 21, iso-hsa-miR-21-5p with SEQ ID NO: 22, iso-hsa-miR-486-5p with SEQ ID NO: 23, iso-hsa-miR-486-5p with SEQ ID NO: 24, iso-hsa-miR-25-3p with SEQ ID NO: 25, iso-hsa-miR-3613-5p with SEQ ID NO: 26, iso-hsa-miR-142-3p with SEQ ID NO: 27, iso-hsa-miR-664a-5p with SEQ ID NO: 28, iso-hsa-miR-151a-3p with SEQ ID NO: 29, iso-hsa-miR-92a-3p with SEQ ID NO: 30, iso-hsa-let-7a-5p with SEQ ID NO: 31, iso-hsa-let-7b-5p with SEQ ID NO: 32, iso-hsa-miR-100-5p with SEQ ID NO: 33, iso-hsa-miR-191-5p with SEQ ID NO: 34, iso-hsa-let-7a-5p with SEQ ID NO: 35, iso-hsa-let-7d-5p with SEQ ID NO: 36.
- Determining the reads number for 17 miRNA biomarkers from the previous step.

Test for the analysis of the identification of the prognostic canonical miRNA and iso-miRNA biomarkers, the Success Test having 99 % accuracy of AUC ROC prediction curve model includes:
- Taking the plasma sample that has been obtained from a woman.
- Isolating all miRNA followed by performing sequence analysis, and data analysis for selected 10 miRNA: iso-hsa-miR-126-3p, iso-hsa-let-7i-5p, iso-hsa-miR-342-3p, iso-hsa-let-7b-5p, iso-hsa-miR-132-3p, iso-hsa-miR-23b-3p, iso-hsa-miR-125a-5p, hsa-miR-584-5p, iso-hsa-miR-127-3p, iso-hsa-miR-151a-3p.
- Determining the reads number for 10 miRNA biomarkers from the previous step.

The non-invasive test by the Success Test predicates the IVF process successfulness at cut off value of 1, while the selected miRNA molecules having value of ≥ 1 predict successful IVF process, and ones having value of < 1 predict unsuccessful IVF process. The cut off value of 5 classifies the selected miRNA molecules having the value of ≥ 5 as the molecules predicting successful IVF process, and ones having the values of < 5 as predicting IVF process failure.

Non-invasive test method via CultMed Test predicts the IVF process successfulness at the cut off value of 1, while miRNA molecules having the value of ≥ 1 predict a high competent (high-quality) embryo, and ones having the value of < 1 predict a low competent (low-quality) embryo.

In the next step of the non-invasive test the identified miRNA molecules form the woman's plasma are arranged according to their significance for the test result: iso-hsa-miR-126-3p, hsa-let-7i-5p, iso-hsa-miR-342-3p, iso-hsa-let-7b-5p, iso-hsa-miR-132-3p, iso-hsa-miR-23b-3p, iso-hsa-miR-125a-5p, iso-hsa-miR-584-5p, iso-hsa-miR-127-3p, iso-hsa-miR-101-3p, iso-hsa-miR-151a-3p, iso-hsa-miR-92a-3p, iso-hsa-let-7b-5p, iso-hsa-miR-589-5p, iso-hsa-miR-652-3p, iso-hsa-miR-1307-3p, iso-hsa-miR-382-5p, iso-hsa-let-7b-5p, iso-hsa-let-7i-5p.

In the next step of the non-invasive test the identified miRNA molecules from the spent blastocyst medium are arranged according to their significance for the test result: iso-hsa-let-7b-5p, iso-hsa-miR-423-5p, iso-hsa-miR-21-5p, iso-hsa-miR-486-5p, iso-hsa-miR-486-5p, iso-hsa-miR-25-3p, iso-hsa-miR-3613-5p, iso-hsa-miR-142-3p, iso-hsa-miR-664a-5p, iso-hsa-miR-151a-3p, iso-hsa-miR-92a-3p, iso-hsa-let-7a-5p, iso-hsa-let-7b-5p, iso-hsa-miR-100-5p, iso-hsa-miR-191-5p, iso-hsa-let-7a-5p, iso-hsa-let-7d-5p.

The advantage of the diagnostic test is the availability of the result for IVF process successfulness/failure within 24 hours from the sampling of the biological material.

The main subjects of the present invention were performed using the method of massive-parallel sequencing of non-coding small RNA molecules (miRNA) at Illumina sequencing platform. On the basis of the specific data analysis (Explanatory Data Analysis) and Machine Learning methods with several models, a number of predictive biomarkers for the individual biological material types were selected.

A list of the particular canonical miRNA and iso-miRNA molecules identified from women plasmas and canonical and iso-miRNA molecules identified from the spent blastocyst media represent another fundamental part of the invention.

miRNA molecules detected by the Success Test are shown in Table 1.

**Table 1 Biomarkers for miRNA prediction of the IVF process successfulness/failure**

| **miRNA biomarkers for the prediction of IVF process successfulness/failure** | **Coef** | **P** |
|---|---|---|
| | (Coefficient of significance) | (p-value, H0: data distribution in relation to IVF result is identical) |
| iso-hsa-miR-126-3p_CGTACCGTGAGAAATAATGCGT | 11.520844189682128 | 0.0016884300209033492 |
| iso-hsa-let-7i-5p_TGTGGTAGTAGTTTGTGCTGTA | 10.851674641148328 | 0.002221447605170916 |
| iso-hsa-mi R-342-3p_TCTCACACAGAAATCGGACCCGTCT | 10.765113798008537 | 0.0023025382184651104 |
| iso-hsa-let-7b-5p_TGAGGTAGTAGGTTGTGTGTTA | 10.547046485537292 | 0.002521143336464067 |
| iso-hsa-miR-132-3p_TAACAGCCTACAGCCATGGTCGT | 9.910931174089068 | 0.0032951920974540376 |
| iso-hsa-mi R-23b-3p_GCACATTGCCAGGGATTACCACT | 9.373481781376519 | 0.0041475542794915604 |
| iso-hsa-miR-125a-5p_TCCCTGAGACCTTTTAACCTGT | 9.18292128014561 | 0.004503999211390216 |
| iso-hsa-mi R-584-5p_TATGGTTTGCCTGGGACTG | 9.094736842105265 | 0.004679908836274879 |
| iso-hsa-miR-127-3p_TCGGATCAGTCTGAGCTTGGCTTTT | 9.094736842105265 | 0.004679908836274879 |
| iso-hsa-miR-101-3p_TACACTACTGTGATAACTGA | 8.845594649607445 | 0.005217803371020453 |
| iso-hsa-miR-151a-3p_TACTAGACTAAAGCTCCTTGAGGT | 8.845594649607445 | 0.005217803371020453 |
| iso-hsa-mi R-92a-3p_GATTGCACTTGTCCCGGCCTGAA | 8.845594649607445 | 0.005217803371020453 |
| iso-hsa-let-7b-5p_TGAGGTAGTATGTTGTGTGG | 8.816195372750641 | 0.005285512415951764 |
| iso-hsa-mi R-589-5p_TGAGAACCACGTCCGCTCTGAGC | 8.696842105263158 | 0.0055702068262932485 |
| iso-hsa-mi R-652-3p_GATGGCGCCACTAGGGTTGTGA | 8.418140050047175 | 0.0063009456943287695 |
| iso-hsa-miR-1307-3p_ACTCGGCGTAGCGTCGGTCGTGT | 8.418140050047175 | 0.0063009456943287695 |
| iso-hsa-mi R-382-5p_GAAGTTGTTCGCGGTGGATTC | 8.289473684210527 | 0.006672365487531239 |
| iso-hsa-let-7b-5p_TGAGGTAGTAAGTTGTGTGGTAT | 8.289473684210527 | 0.006672365487531239 |
| iso-hsa-let-7i-5p_TGAGTTAGTAGTTTGTGCTGTTTT | 8.289473684210527 | 0.006672365487531239 |

miRNAs in Table 1 are ordered according to their significance coefficients, which coefficients determine how important is it to deal with a specific molecule in the analysis (what is its role in the result prediction).

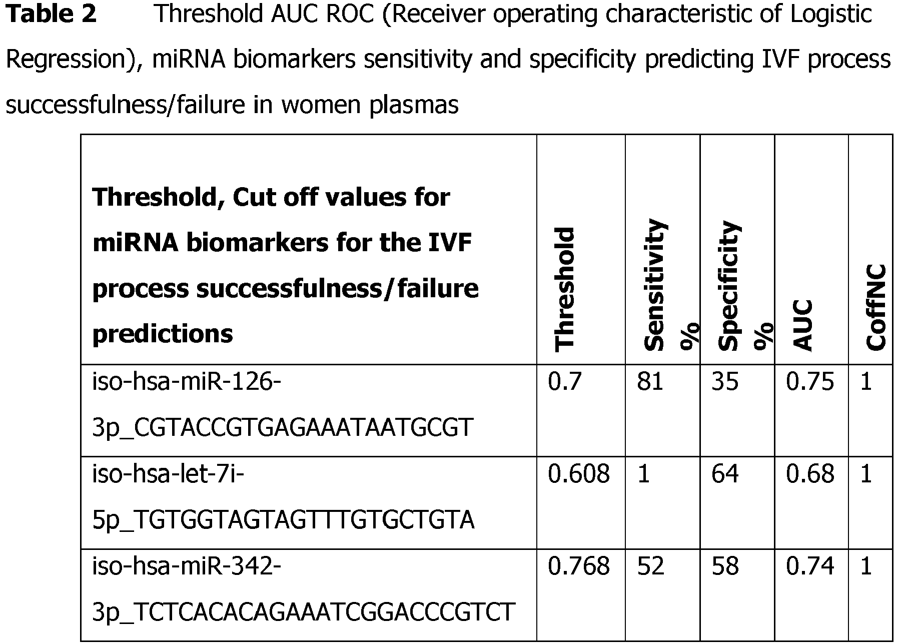

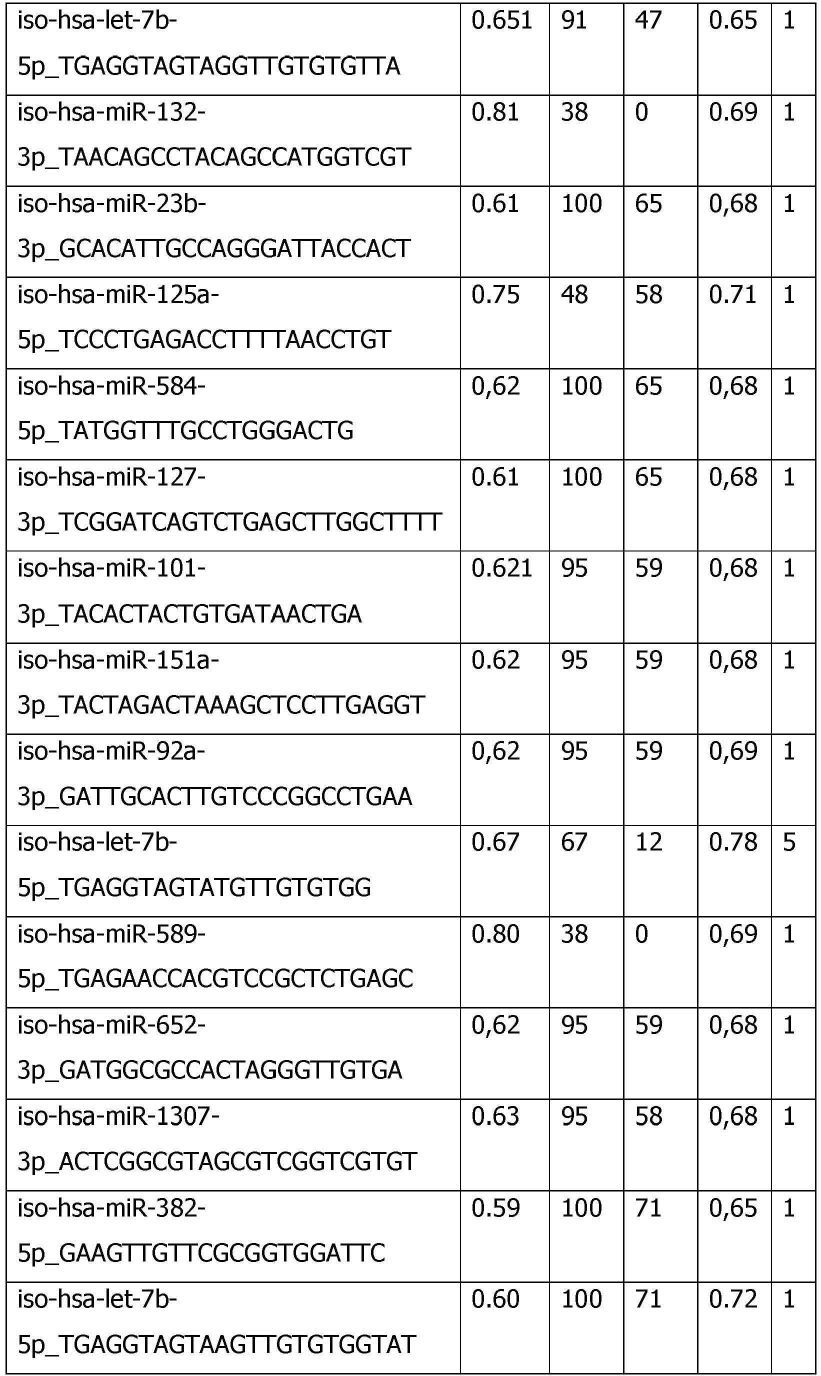

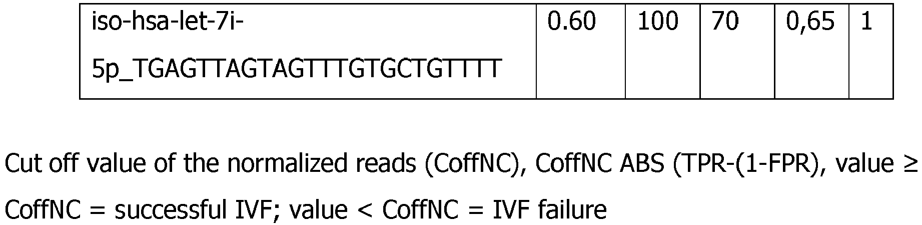

For each of the selected miRNA molecules the minimal number of sequencing reads stated in the last column in Table 2 is needed (cut off value of the normalized reads = 1 or 5). If a miRNA molecule has its cut off value determined as 1, then all values ≥ 1 predict successful IVF process, and miRNA molecules cut off values < 1 predict IVF process failure. The cut off value for the normalized reads determined for iso-hsa-let-7b-5p molecule is 5. It means that any value ≥ 5 predicts a successful IVF process, and any value < 5 predicts IVF process failure.

For 100% prediction of IVF process successfulness/failure it is necessary to analyze each of 19 selected biomarker miRNA molecules (Table 2). Also lower number of miRNA molecules can be used for the analysis of IVF process successfulness/failure, however, the result prediction accuracy will be lower.

The Success Test can be used as safe, non-invasive diagnostic test of IVF process successfulness. It is possible to make a commercial product (service) intended for the public on the basis of this test.

Said canonical and iso-miRNA molecules having the above sequences can be used also in other forms of diagnostic tests as specific probes for the determination of IVF process successfulness.

Table 3 shows the list of the particular canonical and iso-miRNA molecules detected from the spent blastocyst media by the CultMed Test. The molecules identified in the spent blastocyst medium are ordered according their importance, i.e. significance on the basis of the significance coefficient determining how important it is to deal with a given molecule in the analysis (how significant is its role in result prediction).

**Table 3**

| Prediction biomarkers for the selection of competent embryo/incompetent embryo in the IVF process | | |
|---|---|---|
| **Prediction biomarkers for the selection of competent embryo/incompetent embryo in IVF process** | **coef** | **P** |
| | | (p-value, H0: data distribution in relation to IVF result is identical) |
| | (Coefficient of significance) | |
| iso-hsa-let-7b-5p_TGAGGTGGTAGGTTGTGTGGT | 9.621526827042178 | 0.003391088823611076 |
| iso-hsa-miR-423-5p_TGAGGGGCAGAGAGCGAGACTTA | 9.529546351084809 | 0.003532537765767483 |
| iso-hsa-miR-21-5p_TAGCTTACCAGACTGATGTTGAC | 9.37945619335347 | 0.003776937060541304 |
| iso-hsa-mi R-486-5p_TCCTGTACTGAGCTGCCCAGAGA | 8.899093803575798 | 0.004687231604519787 |
| iso-hsa-mi R-486-5p_TCCTGTACTGAGCTGCCTCGAG | 7.886037735849056 | 0.007462265245605795 |
| iso-hsa-mi R-25-3p_CATTGCAATTGTCTCGGTCTGA | 7.166666666666666 | 0.010471649328310581 |
| iso-hsa-mi R-3613-5p_TGTTGTACTTTTTTTTTTGTT | 6.723975915506861 | 0.01294940775142319 |
| iso-hsa-mi R-142-3p_TGTAGTGTTTCCTACCTTATGGA | 6.254545454545453 | 0.0162767044425456 |
| iso-hsa-miR-664a-5p_ACTGGCTAGGGAAAATGATTGGA iso-hsa-miR-151a- | 6.254545454545453 | 0.0162767044425456 |
| 3p_CAAGACTGAAGCTCCTTGAGG | 5.757024793388432 | 0.020828127625493652 |
| iso-hsa-mi R-92a-3p_TATCGCACTTGTCCCGGCCTGT | 5.752285490865144 | 0.020877563509075946 |
| iso-hsa-let-7a-5p_TGATGTAGTAGGTTGTATAG | 5.676567656765675 | 0.021684740586716857 |
| iso-hsa-let-7b-5p_TGAGGTAGTAGGTTGTGTGGTTTA | 5.618093452752898 | 0.022331072190788642 |
| iso-hsa-miR-100-5p_AACCCGTAGATCCGAACTTGT | 5.448383233532933 | 0.024327313909886367 |
| iso-hsa-mi R-191-5p_CAATGGAATCCCAAAAGCAGCTG | 5.3960784313725485 | 0.024980707010107902 |
| iso-hsa-let-7a-5p_TGAGTTAGTAGGTTGTATAG | 5.3960784313725485 | 0.024980707010107902 |
| iso-hsa-let-7d 5p_AGAGGTAGTAGGTTGCGTAGTT | 5.3960784313725485 | 0.024980707010107902 |

17 prediction biomarkers classified as significant prediction biomarkers for the determination of an embryo competence in the IVF process were selected on the basis of the machine learning methods.

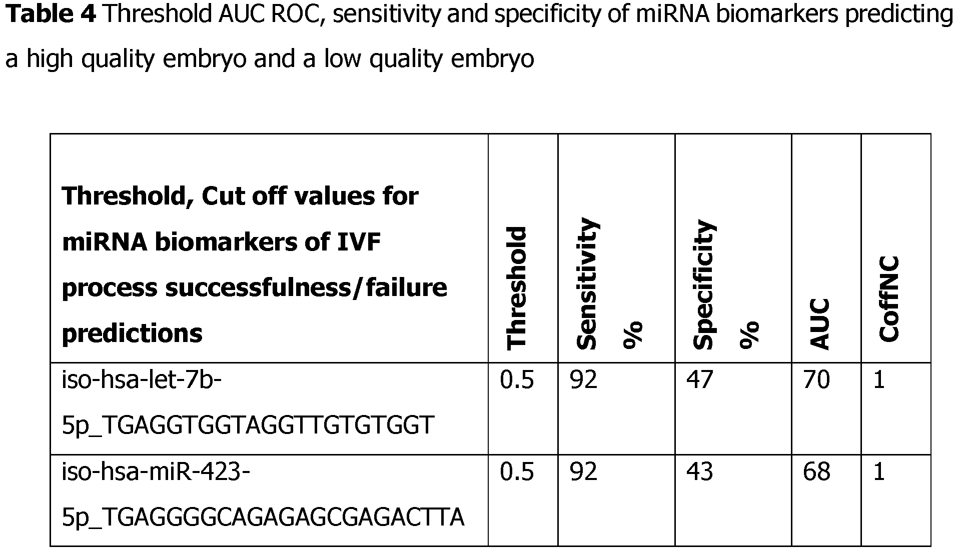

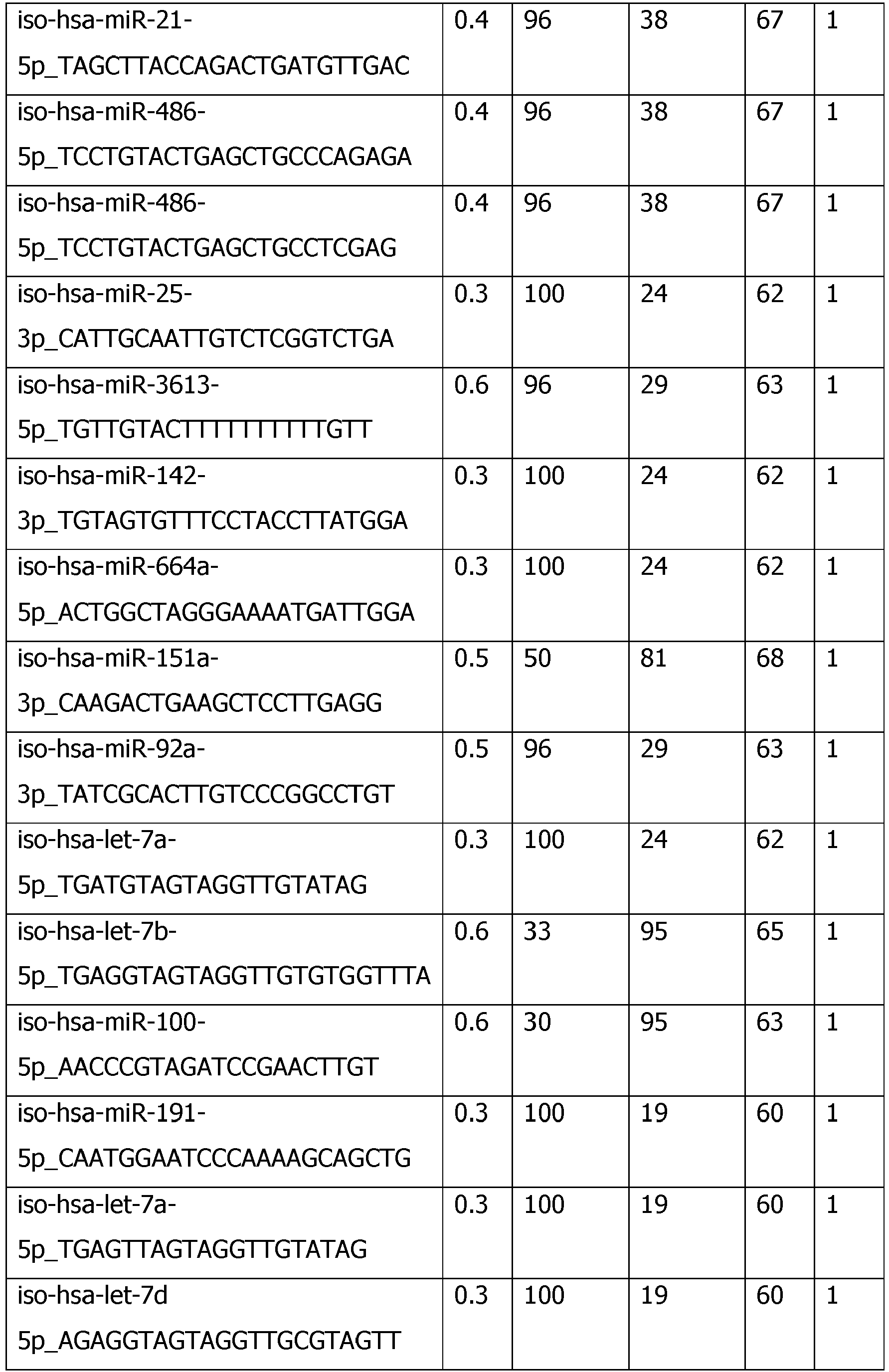

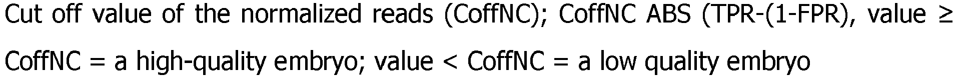

The last column of Table 4 states the cut off value of normalized reads. If a miRNA molecule has its cut off value determined as 1, then all values ≥ 1 predict a high-quality/high competent embryo, and miRNA molecules cut off values < 1 predict a low-quality/low-competent embryo. On the basis of these values it is possible to select a high-quality embryo suitable for the transfer in the IVF process.

All 17 selected miRNA biomarkers are needed for the prediction analysis of high-competent/low-competent embryo with 100% sensitivity and 80% specificity. Also lower number of miRNA molecules can be used for the analysis of a high-competent/low-competent embryo; however, the result prediction accuracy will be lower.

The CultMed Test can be also used as the safe, non-invasive diagnostic test for the selection of a high-competent embryo. This test can be made as a diagnostic product (service); however it can be also used for the selection of an embryo, which is the most suitable for the IVF transfer (as a complementary test to the Success Test).

Said canonical and iso-miRNA molecules having the above sequences can be used also in other forms of diagnostic tests as specific probes for the selection of an embryo, which is the most suitable for the IVF transfer.

The tests are independent and it is suitable to combine them, in order to increase IVF process successfulness, due to the optimization of IVF term prediction in a patient, as well as more effective embryo selection. Said diagnostic test can be offered to infertile couples as a service - a mother competence diagnosis (selection of the most suitable IVF transfer day), an embryo selection.

### Brief Description of Drawings

Figure 1 - Resulting ROC curve based on the analysis of 19 miRNA biomarkers from woman's plasma
Figure 2 - Resulting ROC curve based on the analysis of 10 miRNA biomarkers from woman's plasma
Figure 3 - Resulting ROC curve based on the analysis of 17 miRNA biomarkers from a spent blastocyst medium
Figure 4 - Resulting ROC curve based on the analysis of 14 miRNA biomarkers from a spent blastocyst medium

### Examples of the Embodiment

### Example 1

Example 1 describes the detection of the particular canonical miRNA and iso-miRNA molecules isolated from peripheral blood plasma of 37 women in the age under 37 years by NGS sequencing method. At the day of the embryo transfer, peripheral blood was collected from the women, which blood was used for plasma non-coding RNA molecules analysis. 19 selected canonical miRNA and iso-miRNA molecules were analyzed (Table 2).

### Evaluation:

In the tested plasma the cut off values for miRNA biomarker were at the level ≥ 1. In case of iso-hsa-let-7b-5p biomarker the cut off value was ≥ 5.

The remaining cut off values were < 1.

The successful IVF process was achieved in 20 women and IVF process failure was observed in 17 women.

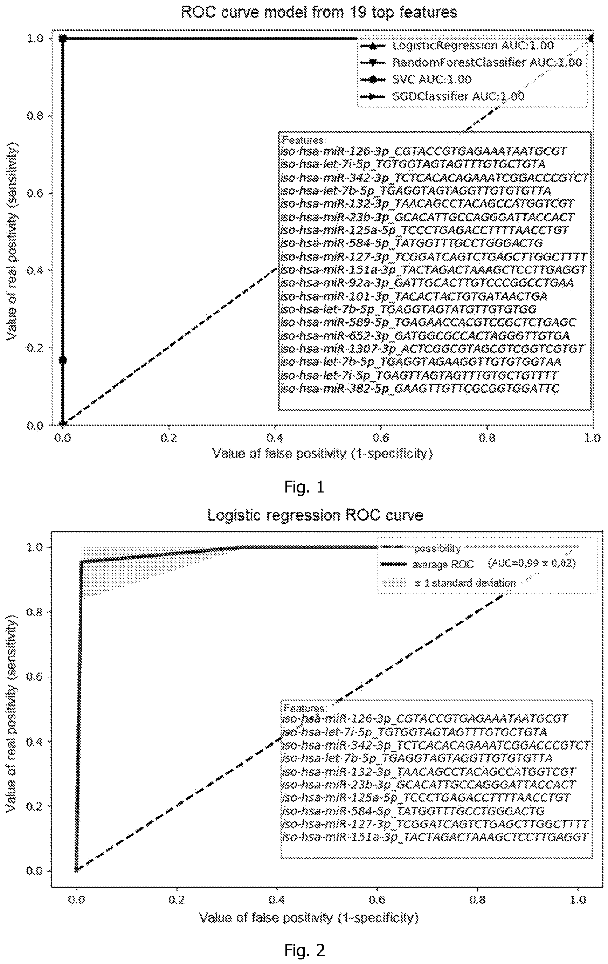

ROC analysis (Receiver Operating Characteristic curve) showed that the combination of 19 canonical miRNA molecules isolated from the women's plasma on the embryo transfer day (see Table 2) predict the IVF process successfulness/failure with 100 % sensitivity and 100 % specificity (Area Under the ROC curve, AUC = 1,0, [1-1]; p = 0.001), as illustrated in Figure 1.

### Example 2

Example 2 describes the detection of the particular canonical miRNA and iso-miRNA molecules detected in plasma of 37 women in the age under 37 years by NGS sequencing method. On the day of the embryo transfer, peripheral blood was collected from the women, whose blood was used for plasma non-coding RNA molecules analysis. Lower number of the canonical miRNA was used for the analysis. 10 canonical miRNA and iso-miRNA as stated in the following table were selected:

| **miRNA biomarkers for the prediction of IVF process successfulness/failure** |
|---|
| iso-hsa-miR-126-3p_CGTACCGTGAGAAATAATGCGT |
| iso hsa-let-7i-5p_TGTGGTAGTAGTTTGTGCTGTA |
| iso-hsa-miR-342-3p_TCTCACACAGAAATCGGACCCGTCT |
| iso-hsa-let-7b-5p_TGAGGTAGTAGGTTGTGTGTTA |
| iso-hsa-miR-132-3p_TAACAGCCTACAGCCATGGTCGT |
| iso-hsa-miR-23b-3p_GCACATTGCCAGGGATTACCACT |
| iso-hsa-miR-125a-5p_{_}TCCCTGAGACCTTTTAACCTFGT |
| iso-hsa-miR-584-5p_TATGGTTTGCCTGGGACTG |
| iso-hsa-miR-127-3p_TCGGATCAGTCTGAGCTTGGCTTTT |
| iso-hsa-miR-151a-3p_TACTAGACTAAAGCTCCTTGAGGT |

### Evaluation:

The method of the test samples and confirmation samples permutation was used in case of the change of the significant biomarkers number, with 7 repetitions and 20/80 percent ratio. ROC method analysis showed that the combination of 10 canonical miRNA molecules collected from the women's plasma on the embryo transfer day (Figure 2) predicts the IVF process successfulness/failure. Average value of the prediction accuracy measured by AUC calculation is at the level of 0.97 (resp. 97 %), with variation +/- 0.06 (Figure 2). The analysis of 10 canonical miRNAs (from plasma) has the prediction accuracy for the test result at the level of 97 %.

### Example 3

Example 3 describes the detection of the particular canonical and iso-hsa-miRNA molecules isolated from the cultivation media of 45 embryos. The cultivation medium was standardly collected on day 5 of the embryo's cultivation. The embryo secreted non-coding RNA molecules to the cultivation fluid, whose molecules are used for the differential selection of a high-quality embryo. 17 iso-hsa-miRNA molecules sequenced by NGS method were selected from a number of the molecules detected in the spent blastocyst medium (Table 4). The number of miRNA biomarkers predicting a high-quality/low-quality embryo was determined by the machine learning methods.

### Evaluation:

The cut off values of the cultivation media of the high-competent embryos were ≥ 1 for all tested miRNA biomarkers.

The cut off values of the cultivation media of the low-competent embryos were < 1 for all tested miRNA biomarkers.

On the basis of the results the spent blastocyst media were divided into two groups: 25 cultivation media showed the high-competent/high-quality embryo, 20 cultivation media showed the low-competent/low-quality embryo.

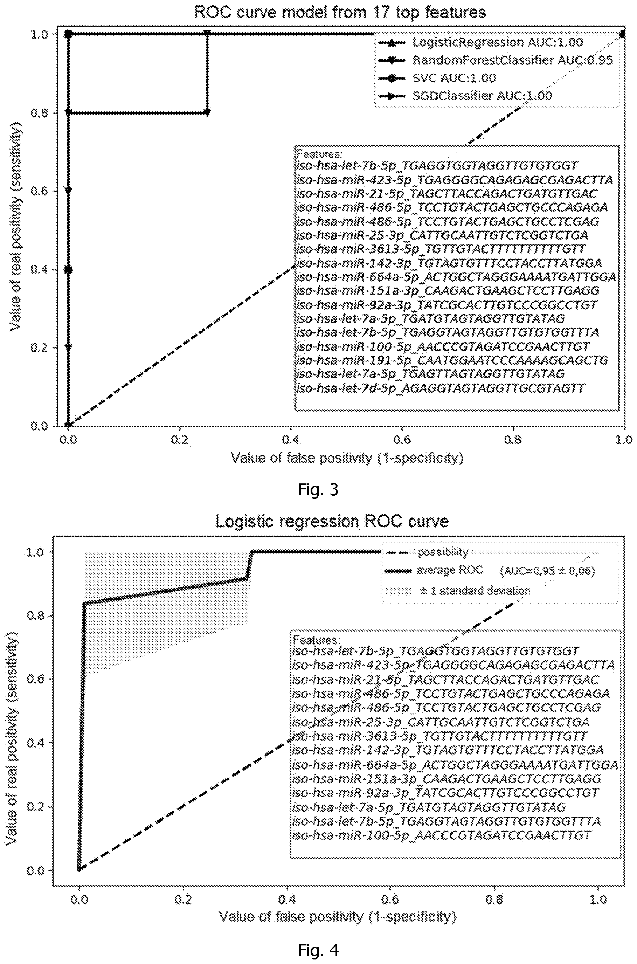

ROC analysis showed that 17 canonical and iso-miRNA molecules (Table 4) distinguish, predict, differentiate a high-competent embryo from a low-competent (non-successful) embryo in the IVF process with 100% sensitivity and 80% specificity (AUC = 0.9 [0.8-1.0]; p=0.001). ROC is illustrated in figure 3.

### Example 4

The protocol was the same as in Example 3, however 14 canonical and iso-miRNA molecules isolated from the spent blastocyst media were selected for the analysis of a high-competent/low-competent embryo, and these molecules are shown in the following table:

| **Prediction biomarkers for the selection of competent/incompetent embryo in IVF process** |
|---|
| iso-hsa-let-7b-5p_TGAGGTGGTAGGTTGTGTGGT |
| iso-hsa-miR-423-5p_TGAGGGGCAGAGAGCGAGACTTA |
| iso-hsa-miR-21-5p_TAGCTTACCAGACTGATGTTGAC |
| iso-hsa-miR-486-5p_TCCTGTACTGAGCTGCCCAGAGA |
| iso-hsa-miR-486-5p_TCCTGTACTGAGCTGCCTCGAG |
| iso-hsa-miR-25-3p_CATTGCAATTGTCTCGGTCTGA |
| iso-hsa-miR-3613-5p_TGTTGTACTTTTTTTTTTGTT |
| iso-hsa-miR-142-3p_TGTAGTGTTTCCTACCTTATGGA |
| iso-hsa-miR-664a-5p_ACTGGCTAGGGAAAATGATTGGA |
| iso-hsa-miR-151a-3p_CAAGACTGAAGCTCCTTGAGG |
| iso-hsa-miR-92a-3p_TATCGCACTTGTCCCGGCCTGT |
| iso-hsa-let-7a-5p_TGATGTAGTAGGTTGTATAG |
| iso-hsa-let-7b-5p_TGAGGTAGTAGGTTGTGTGGTTTA |
| iso-hsa-miR-100-5p_AACCCGTAGATCCGAACTTGT |

### Evaluation:

As in Example 2, the method of the test samples and confirmation samples permutation was used with 7 repetitions and 20/80 percent ratio. Said ROC analysis method showed that the canonical and iso-miRNA molecules illustrated in Figure 4 distinguish, differentiate a high-competent embryo from a low-competent (non-successful) embryo in the IVF process with lower number of the prediction mRNA biomarkers (14). Average value of the prediction accuracy measured by AUC calculation is at the level of 0.97 (resp. 97 %), with variation +/- 0.06 (Figure 4). The analysis of 14 canonical and iso-miRNAs from the spent blastocyst medium provides the prediction accuracy for the test result at the level of 97 %.

### Literature

- de Mouzon, Hazout, Cohen-Bacrie, Belloc, Cohen-Bacrie, Blood type and ovarian reserve, Human Reproduction, 27, (5), 2012,1544-1545.
- Kosteria , Anagnostopoulos , Kanaka-Gantenbein , Chrousos , The Use of Proteomics in Assisted Reproduction, In Vivo, 31(3), 2017, 267-283.
- Asirvatham, Magner, miRNA regulation of cytokine genes, Cytokine, 45, (2), 2009, 58-69.
- McCallie , Schoolcraft , Katz-Jaffe MG, Aberration of blastocyst microRNA expression is associated with human infertility, Fertil Steril, 93(7), 2010, 2374-82.
- Mouillet, Ouyang, Coyne, MicroRNAs in placental health and disease, American Journal of Obstetrics and Gynecology 213, 2015, 163-S172.
- Catalanotto, Cogoni, Zardo, MicroRNA in control of gene expression: an overview of nuclear functions. International Journal of Molecular Sciences, 17, 2016.
- Rosenbluth, Shelton, Sparks, Devor, Christenson, Van Voorhis, MicroRNA expression in the human blastocyst, Fertil Steril., 1, 99(3), 2013, 855-861.
- Lee, Finniss, Cazacu, Xiang, Brodie, Mesenchymal stem cells deliver exogenous miRNAs to neural cells and induce their differentiation and glutamate transporter expression, Stem Cells Dev., 1,(23), 2014 Dec 2851-61.
- Chang, Wakeland, Trophoblast lineage specification, differentiation and their regulation by oxygen tension. Journal of Endocrinology 236, 2018,43-R56.
- Tang, Kaneda, O'Carroll, Hajkova, Barton, Sun, Lee, Tarakhovsky, Lao, Surani, Maternal microRNAs are essential for mouse zygotic development, Genes Dev., 21(6), 2007, 644-8.
- Zhang, Dunk, Croy To serve and to protect: the role of decidual innate immune cells on human pregnancy. Cell and Tissue Research, 363, 2016 249-265.
- Bonilla-Musoles, Raga, Osborne, Castillo, Bonilla, Endometrial receptivity: evaluation with ultrasound, Ultrasound Q., 29(1), 2013, 3-20.

## Claims

1. A method for the evaluation of *in vitro* fertilization process successfulness, **characterized in that** it comprises two independent analyses:
- Identification and analysis of selected prognostic canonical miRNA and/or iso-miRNA biomarkers from women's peripheral blood plasma on the day of the *in vitro* fertilization process;
wherein successful evaluation shows cut off values ≥ 1 at reads number of 1, or cut off values ≥ 5 at reads number of 5;
- Identification and analysis of selected prognostic canonical miRNAs and/or iso-miRNAs from a spent blastocyst medium collected once on day 4 to 6 of the embryo's cultivation with 95% prediction accuracy established by AUC method comprises the following steps:
i. Taking the spent blastocyst medium sample;
ii. Isolating all miRNA and/or iso-miRNA biomarkers, followed by performing sequence analysis, and the data analysis for the selected 14 miRNA and/or iso-miRNA biomarkers ordered according to their significance for the *in vitro* fertilization process result:
iso-hsa-let-7b-5p v SEQ ID NO: 20, TGAGGTGGTAGGTTGTGTGGT
iso-hsa-miR-423-5p in SEQ ID NO: 21, TGAGGGGCAGAGAGCGAGACTTA
iso-hsa-miR-21-5p in SEQ ID NO: 22, TAGCTTACCAGACTGATGTTGAC
iso-hsa-miR-486-5p in SEQ ID NO: 23, TCCTGTACTGAGCTGCCCAGAGA
iso-hsa-miR-486-5p in SEQ ID NO: 24, TCCTGTACTGAGCTGCCTCGAG
iso-hsa-miR-25-3p in SEQ ID NO: 25, CATTGCAATTGTCTCGGTCTGA
iso-hsa-miR-3613-5p in SEQ ID NO: 26, TGTTGTACTTTTTTTTTTGTT
iso-hsa-miR-142-3p in SEQ ID NO: 27, TGTAGTGTTTCCTACCTTATGGA
iso-hsa-miR-664a-5p in SEQ ID NO: 28, ACTGGCTAGGGAAAATGATTGGA
iso-hsa-miR-151a-3p in SEQ ID NO: 29, CAAGACTGAAGCTCCTTGAGG
iso-hsa-miR-92a-3p in SEQ ID NO: 30, TATCGCACTTGTCCCGGCCTGT
iso-hsa-let-7a-5p in SEQ ID NO: 31, TGATGTAGTAGGTTGTATAG
iso-hsa-let-7b-5p in SEQ ID NO: 32, TGAGGTAGTAGGTTGTGTGGTTTA
iso-hsa-miR-100-5p in SEQ ID NO: 33, AACCCGTAGATCCGAACTTGT
iii. Determining the reads number for 14 miRNA biomarkers from step ii);
wherein the successful evaluation shows cut off values ≥ 1 at reads number of 1; and
wherein if the evaluation of both analyses is successful, then also the prediction of *in vitro* fertilization process is successful.

2. The method according to claim 1, **characterized in that** the evaluation of the identification of the selected prognostic canonical miRNA and iso-miRNA biomarkers from the women's peripheral blood plasma on the day of the *in vitro* fertilization process with 97% prediction accuracy established by AUC method comprises the following steps:
a) Taking a peripheral blood plasma sample that has been obtained from a woman;
b) Isolating all miRNAs, followed by performing sequence analysis, and data analysis for the selected 10 miRNA and/or iso-miRNA biomarkers ordered according to their significance for the *in vitro* fertilization process result:
iso-hsa-miR-126-3p in SEQ ID NO: 1, CGTACCGTGAGAAATAATGCGT
iso-hsa-let-7i-5p in SEQ ID NO: 2, TGTGGTAGTAGTTTGTGCTGTA
iso-hsa-miR-342-3p in SEQ ID NO: 3, TCTCACACAGAAATCGGACCCGTCT
iso-hsa-let-7b-5p in SEQ ID NO: 4, TGAGGTAGTAGGTTGTGTGTTA
iso-hsa-miR-132-3p in SEQ ID NO: 5, TAACAGCCTACAGCCATGGTCGT
iso-hsa-miR-23b-3p in SEQ ID NO: 6, GCACATTGCCAGGGATTACCACT
iso-hsa-miR-125a-5p in SEQ ID NO: 7, TCCCTGAGACCTTTTAACCTGT
iso-hsa-miR-584-5p in SEQ ID NO: 8, TATGGTTTGCCTGGGACTG
iso-hsa-miR-127-3p in SEQ ID NO: 9, TCGGATCAGTCTGAGCTTGGCTTTT
iso-hsa-miR-151a-3p in SEQ ID NO:11, TACTAGACTAAAGCTCCTTGAGGT
c) Determining the reads number for 10 miRNA biomarkers from step b).

3. The method according to claim 1, **characterized in that** the evaluation of the identification of the selected prognostic canonical miRNA and iso-miRNA biomarkers from the women's peripheral blood plasma on the day of the *in vitro* fertilization process with 100% sensitivity and 100% specificity comprises the following steps:
a) Taking a peripheral blood plasma sample that has been obtained from a woman;
b) Isolating all miRNAs, followed by performing sequence analysis, and the data analysis for the selected 19 miRNAs ordered according to their significance for the *in vitro* fertilization process result:
iso-hsa-miR-126-3p in SEQ ID NO: 1, CGTACCGTGAGAAATAATGCGT
iso-hsa-let-7i-5p in SEQ ID NO: 2, TGTGGTAGTAGTTTGTGCTGTA
iso-hsa-miR-342-3p in SEQ ID NO: 3, TCTCACACAGAAATCGGACCCGTCT
iso-hsa-let-7b-5p in SEQ ID NO: 4, TGAGGTAGTAGGTTGTGTGTTA
iso-hsa-miR-132-3p in SEQ ID NO: 5, TAACAGCCTACAGCCATGGTCGT
iso-hsa-miR-23b-3p in SEQ ID NO: 6, GCACATTGCCAGGGATTACCACT
iso-hsa-miR-125a-5p in SEQ ID NO: 7, TCCCTGAGACCTTTTAACCTGT
iso-hsa-miR-584-5p in SEQ ID NO: 8, TATGGTTTGCCTGGGACTG
iso-hsa-miR-127-3p in SEQ ID NO: 9, TCGGATCAGTCTGAGCTTGGCTTTT
iso-hsa-miR-101-3p in SEQ ID NO: 10, TACACTACTGTGATAACTGA
iso-hsa-miR-151a-3p in SEQ ID NO:11, TACTAGACTAAAGCTCCTTGAGGT
iso-hsa-miR-92a-3p in SEQ ID NO: 12, GATTGCACTTGTCCCGGCCTGAA
iso-hsa-let-7b-5p in SEQ ID NO: 13, TGAGGTAGTATGTTGTGTGG
iso-hsa-miR-589-5p in SEQ ID NO: 14, TGAGAACCACGTCCGCTCTGAGC
iso-hsa-miR-652-3p in SEQ ID NO: 15, GATGGCGCCACTAGGGTTGTGA
iso-hsa-miR-1307-3p in SEQ ID NO: 16, ACTCGGCGTAGCGTCGGTCGTGT
iso-hsa-miR-382-5p in SEQ ID NO: 17, GAAGTTGTTCGCGGTGGATTC
iso-hsa-let-7b-5p in SEQ ID NO: 18, TGAGGTAGTAAGTTGTGTGGTAT
iso-hsa-let-7i-5p in SEQ ID NO: 19, TGAGTTAGTAGTTTGTGCTGTTTT
c) Determining the reads number for all 19 miRNA and/or iso-miRNA biomarkers from step b).

4. The method according to claim 1, **characterized in that** the evaluation of the identification of the selected prognostic canonical miRNA and iso-miRNA biomarkers from the spent blastocyst medium with 100% sensitivity and 80% specificity comprises the following steps:
i. Taking the spent blastocyst medium sample;
ii. Isolating all miRNA and/or iso-miRNA biomarkers, followed by performing sequence analysis, and the data analysis for the selected 17 miRNA and/or iso-miRNA biomarkers ordered according to their significance for the *in vitro* fertilization process result:
iso-hsa-let-7b-5p in SEQ ID NO: 20, TGAGGTGGTAGGTTGTGTGGT
iso-hsa-miR-423-5p in SEQ ID NO: 21, TGAGGGGCAGAGAGCGAGACTTA
iso-hsa-miR-21-5p in SEQ ID NO: 22, TAGCTTACCAGACTGATGTTGAC
iso-hsa-miR-486-5p in SEQ ID NO: 23, TCCTGTACTGAGCTGCCCAGAGA
iso-hsa-miR-486-5p in SEQ ID NO: 24, TCCTGTACTGAGCTGCCTCGAG
iso-hsa-miR-25-3p in SEQ ID NO: 25, CATTGCAATTGTCTCGGTCTGA
iso-hsa-miR-3613-5p in SEQ ID NO: 26, TGTTGTACTTTTTTTTTTGTT
iso-hsa-miR-142-3p in SEQ ID NO: 27, TGTAGTGTTTCCTACCTTATGGA
iso-hsa-miR-664a-5p in SEQ ID NO: 28, ACTGGCTAGGGAAAATGATTGGA
iso-hsa-miR-151a-3p in SEQ ID NO: 29, CAAGACTGAAGCTCCTTGAGG
iso-hsa-miR-92a-3p in SEQ ID NO: 30, TATCGCACTTGTCCCGGCCTGT
iso-hsa-let-7a-5p in SEQ ID NO: 31, TGATGTAGTAGGTTGTATAG
iso-hsa-let-7b-5p in SEQ ID NO: 32, TGAGGTAGTAGGTTGTGTGGTTTA
iso-hsa-miR-100-5p in SEQ ID NO: 33, AACCCGTAGATCCGAACTTGT
iso-hsa-miR-191-5p in SEQ ID NO: 34, CAATGGAATCCCAAAAGCAGCTG
iso-hsa-let-7a-5p in SEQ ID NO: 35, TGAGTTAGTAGGTTGTATAG
iso-hsa-let-7d 5p in SEQ ID NO: 36 AGAGGTAGTAGGTTGCGTAGTT
iii. Determining the reads number for all 17 miRNA and/or iso-miRNA biomarkers from step ii).

5. The method according to claims 2 or 3, **characterized in that** the cut off value of 1 for the selected prognostic canonical miRNA and/or iso-miRNA biomarkers from women's peripheral blood plasma on the day of the *in vitro* fertilization process classifying the miRNA molecules having the value ≥ 1 to be the molecules predicting successful IVF process, and those having the value < 1 to be the molecules predicting IVF process failure, and the cut off value of 5 classifying miRNA molecules having the value ≥ 5 to be the molecules predicting successful *in vitro* fertilization process and those having the value < 5 to be the molecules predicting the *in vitro* fertilization process failure.

6. The method according to claims 1 or 4, **characterized in that**, the cut off value of 1 for the selected prognostic canonical miRNA and/or iso-miRNA from the spent blastocyst medium collected once on day 4 to 6 of the embryo's cultivation classifies miRNAs having the value ≥ 1 to be the molecules predicting a high-competent (high-quality) embryo, and thus the successful *in vitro* fertilization process, and the values < 1 predict a low-competent (low-quality) embryo and thus the *in vitro* fertilization process failure.

## Patentansprüche

1. Verfahren zur Bewertung des Erfolges des *In-vitro*-Fertilisationsprozesses, **dadurch gekennzeichnet, dass** es zwei unabhängige Analysen umfasst:
- Identifizierung und Analyse ausgewählter prognostischer kanonischer miRNA- und/oder iso-miRNA-Biomarker aus dem peripheren Blutplasma der Frauen am Tag des *In-vitro*-Fertilisationsprozesses;
wobei eine erfolgreiche Auswertung Grenzwerte ≥ 1 bei einer Anzahl der Lesungen 1 oder Grenzwerte ≥ 5 bei einer Anzahl der Lesungen 5 aufweist;
- Identifizierung und Analyse ausgewählter prognostischer kanonischer miRNAs und/oder iso-miRNAs aus einem Embryokulturmedium, das einmal am Tag 4 bis 6 der Kultivierung des Embryos gesammelt wurde, mit einer Vorhersagegenauigkeit von 95%, die mit der AUC-Methode ermittelt wurde, umfasst die folgenden Schritte:
i. Entnahme der Probe des Embryokulturmediums
ii. Isolierung aller miRNA- und/oder iso-miRNA-Biomarker, gefolgt von der Durchführung einer Sequenzanalyse und der Datenanalyse für die ausgewählten 14 miRNA- und/oder iso-miRNA-Biomarker, geordnet nach ihrer Bedeutung für das Ergebnis des *In-vitro*-Fertilisationsprozesses:
iso-hsa-let-7b-5p in SEQ ID NO: 20, TGAGGTGGTAGGTTGTGTGGT
iso-hsa-miR-423-5p in SEQ ID NO: 21, TGAGGGGCAGAGAGCGAGACTTA
iso-hsa-miR-21-5p in SEQ ID NO: 22, TAGCTTACCAGACTGATGTTGAC
iso-hsa-miR-486-5p in SEQ ID NO: 23, TCCTGTACTGAGCTGCCCAGAGA
iso-hsa-miR-486-5p in SEQ ID NO: 24, TCCTGTACTGAGCTGCCTCGAG
iso-hsa-miR-25-3p in SEQ ID NO: 25, CATTGCAATTGTCTCGGTCTGA
iso-hsa-miR-3613-5p in SEQ ID NO: 26, TGTTGTACTTTTTTTTTTGTT
iso-hsa-miR-142-3p in SEQ ID NO: 27, TGTAGTGTTTCCTACCTTATGGA
iso-hsa-miR-664a-5p in SEQ ID NO: 28, ACTGGCTAGGGAAAATGATTGGA
iso-hsa-miR-151a-3p in SEQ ID NO: 29, CAAGACTGAAGCTCCTTGAGG
iso-hsa-miR-92a-3p in SEQ ID NO: 30, TATCGCACTTGTCCCGGCCTGT
iso-hsa-let-7a-5p in SEQ ID NO: 31, TGATGTAGTAGGTTGTATAG
iso-hsa-let-7b-5p in SEQ ID NO: 32, TGAGGTAGTAGGTTGTGTGGTTTA
iso-hsa-miR-100-5p in SEQ ID NO: 33, AACCCGTAGATCCGAACTTGT
iii. Bestimmung der Anzahl der Lesungen für 14 miRNA-Biomarker aus Schritt ii);
wobei eine erfolgreiche Auswertung Grenzwerte ≥ 1 bei einer Anzahl der Lesungen 1 aufweist;
und
wobei, wenn die Auswertung beider Analysen erfolgreich ist, auch die Vorhersage des *In-vitro*-Fertilisationsprozesses erfolgreich ist.

2. Verfahren nach Anspruch 1, **dadurch gekennzeichnet, dass** auch die Auswertung der Identifizierung ausgewählter prognostischer kanonischer miRNA- und iso-miRNA-Biomarker aus dem peripheren Blutplasma der Frauen am Tag des *In-vitro-*Fertilisationsprozesses, mit einer Vorhersagegenauigkeit von 97 %, die mit der AUC-Methode ermittelt wurde, umfasst die folgenden Schritte:
a) Entnahme einer peripheren Blutplasmaprobe, die einer Frau entnommen wurde;
b) Isolierung aller miRNAs, gefolgt von der Durchführung einer Sequenzanalyse und einer Datenanalyse für die ausgewählten 10 miRNA- und/oder iso-miRNA-Biomarker, geordnet nach ihrer Bedeutung für das Ergebnis des *In-vitro-*Fertilisationsprozesses:
iso-hsa-miR-126-3p in SEQ ID NO: 1, CGTACCGTGAGAAATAATGCGT
iso-hsa-let-7i-5p in SEQ ID NO: 2, TGTGGTAGTAGTTTGTGCTGTA
iso-hsa-miR-342-3p in SEQ ID NO: 3, TCTCACACAGAAATCGGACCCGTCT
iso-hsa-let-7b-5p in SEQ ID NO: 4, TGAGGTAGTAGGTTGTGTGTTA
iso-hsa-miR-132-3p in SEQ ID NO: 5, TAACAGCCTACAGCCATGGTCGT
iso-hsa-miR-23b-3p in SEQ ID NO: 6, GCACATTGCCAGGGATTACCACT
iso-hsa-miR-125a-5p in SEQ ID NO: 7, TCCCTGAGACCTTTTAACCTGT
iso-hsa-miR-584-5p in SEQ ID NO: 8, TATGGTTTGCCTGGGACTG
iso-hsa-miR-127-3p in SEQ ID NO: 9, TCGGATCAGTCTGAGCTTGGCTTTT
iso-hsa-miR-151a-3p in SEQ ID NO:11, TACTAGACTAAAGCTCCTTGAGGT
c) Bestimmung der Anzahl der Lesungen für 10 miRNA-Biomarker aus Schritt b).

3. Verfahren nach Anspruch 1, **dadurch gekennzeichnet, dass** die Auswertung der Identifizierung ausgewählter prognostischer kanonischer miRNA- und iso-miRNA-Biomarker aus dem peripheren Blutplasma der Frauen am Tag des *In-vitro-*Fertilisationsprozesses mit 100 % Sensitivität und 100 % Spezifität umfasst die folgenden Schritte:
a) Entnahme einer peripheren Blutplasmaprobe, die einer Frau entnommen wurde;
b) Isolierung aller miRNAs, gefolgt von der Durchführung einer Sequenzanalyse und einer Datenanalyse für die ausgewählten 19 miRNAs, geordnet nach ihrer Bedeutung für das Ergebnis des *In-vitro*-Fertilisationsprozesses:
iso-hsa-miR-126-3p in SEQ ID NO: 1, CGTACCGTGAGAAATAATGCGT
iso-hsa-let-7i-5p in SEQ ID NO: 2, TGTGGTAGTAGTTTGTGCTGTA
iso-hsa-miR-342-3p in SEQ ID NO: 3, TCTCACACAGAAATCGGACCCGTCT
iso-hsa-let-7b-5p in SEQ ID NO: 4, TGAGGTAGTAGGTTGTGTGTTA
iso-hsa-miR-132-3p in SEQ ID NO: 5, TAACAGCCTACAGCCATGGTCGT
iso-hsa-miR-23b-3p in SEQ ID NO: 6, GCACATTGCCAGGGATTACCACT
iso-hsa-miR-125a-5p in SEQ ID NO: 7, TCCCTGAGACCTTTTAACCTGT
iso-hsa-miR-584-5p in SEQ ID NO: 8, TATGGTTTGCCTGGGACTG
iso-hsa-miR-127-3p in SEQ ID NO: 9, TCGGATCAGTCTGAGCTTGGCTTTT
iso-hsa-miR-101-3p in SEQ ID NO: 10, TACACTACTGTGATAACTGA
iso-hsa-miR-151a-3p in SEQ ID NO:11, TACTAGACTAAAGCTCCTTGAGGT
iso-hsa-miR-92a-3p in SEQ ID NO: 12, GATTGCACTTGTCCCGGCCTGAA
iso-hsa-let-7b-5p in SEQ ID NO: 13, TGAGGTAGTATGTTGTGTGG
iso-hsa-miR-589-5p in SEQ ID NO: 14, TGAGAACCACGTCCGCTCTGAGC
iso-hsa-miR-652-3p in SEQ ID NO: 15, GATGGCGCCACTAGGGTTGTGA
iso-hsa-miR-1307-3p in SEQ ID NO: 16, ACTCGGCGTAGCGTCGGTCGTGT
iso-hsa-miR-382-5p in SEQ ID NO: 17, GAAGTTGTTCGCGGTGGATTC
iso-hsa-let-7b-5p in SEQ ID NO: 18, TGAGGTAGTAAGTTGTGTGGTAT
iso-hsa-let-7i-5p in SEQ ID NO: 19, TGAGTTAGTAGTTTGTGCTGTTTT
c) Bestimmung der Anzahl der Lesungen aller 19 miRNA- und/oder iso-miRNA-Biomarker aus Schritt b).

4. Verfahren nach Anspruch 1, **dadurch gekennzeichnet, dass** die Auswertung der Identifizierung ausgewählter prognostischer kanonischer miRNA- und iso-miRNA-Biomarker aus einem Embryokulturmedium mit 100 % Sensitivität und 80 % Spezifität umfasst die folgenden Schritte:
i. Entnahme der Probe des Embryokulturmediums;
ii. Isolierung aller miRNA- und/oder iso-miRNA-Biomarker, gefolgt von der Durchführung einer Sequenzanalyse und einer Datenanalyse für die ausgewählten 17 miRNA- und/oder iso-miRNA-Biomarker, geordnet nach ihrer Bedeutung für das Ergebnis des *In-vitro*-Fertilisationsprozesses:
iso-hsa-let-7b-5p in SEQ ID NO: 20, TGAGGTGGTAGGTTGTGTGGT
iso-hsa-miR-423-5p in SEQ ID NO: 21, TGAGGGGCAGAGAGCGAGACTTA
iso-hsa-miR-21-5p in SEQ ID NO: 22, TAGCTTACCAGACTGATGTTGAC
iso-hsa-miR-486-5p in SEQ ID NO: 23, TCCTGTACTGAGCTGCCCAGAGA
iso-hsa-miR-486-5p in SEQ ID NO: 24, TCCTGTACTGAGCTGCCTCGAG
iso-hsa-miR-25-3p in SEQ ID NO: 25, CATTGCAATTGTCTCGGTCTGA
iso-hsa-miR-3613-5p in SEQ ID NO: 26, TGTTGTACTTTTTTTTTTGTT
iso-hsa-miR-142-3p in SEQ ID NO: 27, TGTAGTGTTTCCTACCTTATGGA
iso-hsa-miR-664a-5p in SEQ ID NO: 28, ACTGGCTAGGGAAAATGATTGGA
iso-hsa-miR-151a-3p in SEQ ID NO: 29, CAAGACTGAAGCTCCTTGAGG
iso-hsa-miR-92a-3p in SEQ ID NO: 30, TATCGCACTTGTCCCGGCCTGT
iso-hsa-let-7a-5p in SEQ ID NO: 31, TGATGTAGTAGGTTGTATAG
iso-hsa-let-7b-5p in SEQ ID NO: 32, TGAGGTAGTAGGTTGTGTGGTTTA
iso-hsa-miR-100-5p in SEQ ID NO: 33, AACCCGTAGATCCGAACTTGT
iso-hsa-miR-191-5p in SEQ ID NO: 34, CAATGGAATCCCAAAAGCAGCTG
iso-hsa-let-7a-5p in SEQ ID NO: 35, TGAGTTAGTAGGTTGTATAG
iso-hsa-let-7d 5p in SEQ ID NO: 36 AGAGGTAGTAGGTTGCGTAGTT
iii. Bestimmung der Anzahl der Lesungen aller 17 miRNA- und/oder iso-miRNA-Biomarker aus Schritt ii).

5. Verfahren nach Anspruch 2 oder 3, **dadurch gekennzeichnet, dass** der Grenzwert von 1 für ausgewählte prognostische kanonische miRNA- und/oder iso-miRNA-Biomarker aus dem peripheren Blutplasma der Frauen am Tag des *In-vitro-*Fertilisationsprozesses die miRNA-Moleküle, die den Wert ≥ 1 haben, als Moleküle klassifiziert, die einen erfolgreichen IVF-Prozess vorhersagen, und diejenigen mit dem Wert < 1 als Moleküle klassifiziert, die das Scheitern des IVF-Prozesses vorhersagen, und der Grenzwert von 5, der die miRNA-Moleküle, die den Wert ≥ 5 haben, als Moleküle klassifiziert, die einen erfolgreichen *In-vitro*-Fertilisationsprozess vorhersagen, und diejenigen mit dem Wert < 5 als Moleküle klassifiziert, die das Scheitern des *In-vitro*-Fertilisationsprozesses vorhersagen.

6. Verfahren nach Anspruch 1 oder 4, **dadurch gekennzeichnet, dass** der Grenzwert von 1 für ausgewählte prognostische kanonische miRNAs und/oder iso-miRNAs aus einem Embryokulturmedium, das einmal am Tag 4 bis 6 der Kultivierung des Embryos gesammelt wurde, miRNAs mit dem Wert ≥ 1 als Moleküle klassifiziert, die einen hochkompetenten (qualitativ hochwertigen) Embryo vorhersagen, und damit einen erfolgreichen *In-vitro*-Fertilisationsprozess, während die Werte < 1 einen wenig kompetenten (minderwertigen) Embryo und damit ein Scheitern des *In-vitro-*Fertilisationsprozesses vorhersagen.

## Revendications

1. Méthode d'évaluation de la réussite du processus de fécondation *in vitro,* **caractérisée par le fait qu'**elle consiste à réaliser deux analyses indépendantes:
- L'identification et l'analyse des miARN canoniques et/ou des iso-miARN choisis en tant que les biomarqueurs pronostiques, détectés dans le plasma obtenu à partir d'un échantillon de sang périphérique de la femme prélevé au jour de la réalisation du processus de fécondation *in vitro;*
dans laquelle le résultat de l'évaluation est considéré comme positif si les valeurs seuils ≥ 1 pour le nombre de lectures de 1, ou
si les valeurs seuils ≥ 5 pour le nombre de lectures 5;
- L'identification et l'analyse des miARN canoniques et/ou des iso-miARN choisis en tant que des biomarqueurs pronostiques, détectés dans l'échantillon du milieu utilisé pour la culture de blastocyste et prélevé au jour 4 à 6 de la culture de l'embryon, avec une précision de prédiction de 95 % établie par la méthode ASC, comprennent les étapes suivantes:
i. Prélèvement d'un échantillon du milieu utilisé pour la culture de blastocyste;
ii. Isolement de l'ensemble des miARN et/ou des iso-miARN servant de biomarqueurs, suivi d'une analyse des séquences et d'une analyse des données pour les 14 miARN et/ou iso-miARN choisis comme biomarqueurs, classés en fonction de leur importance pour le résultat du processus de fécondation *in vitro:*
iso-hsa-let-7b-5p dans SEQ ID N° : 20, TGAGGTGGTAGGTTGTGTGGT
iso-hsa-miR-423-5p dans SEQ ID N° : 21, TGAGGGGCAGAGAGCGAGACTTA
iso-hsa-miR-21-5p dans SEQ ID N° : 22, TAGCTTACCAGACTGATGTTGAC
iso-hsa-miR-486-5p dans SEQ ID N° : 23, TCCTGTACTGAGCTGCCCAGAGA
iso-hsa-miR-486-5p dans SEQ ID N° : 24, TCCTGTACTGAGCTGCCTCGAG
iso-hsa-miR-25-3p dans SEQ ID N° : 25, CATTGCAATTGTCTCGGTCTGA
iso-hsa-miR-3613-5p dans SEQ ID N° : 26, TGTTGTACTTTTTTTTTTGTT
iso-hsa-miR-142-3p dans SEQ ID N° : 27, TGTAGTGTTTCCTACCTTATGGA
iso-hsa-miR-664a-5p dans SEQ ID N° : 28, ACTGGCTAGGGAAAATGATTGGA
iso-hsa-miR-151a-3p dans SEQ ID N° : 29, CAAGACTGAAGCTCCTTGAGG
iso-hsa-miR-92a-3p dans SEQ ID N° : 30, TATCGCACTTGTCCCGGCCTGT
iso-hsa-let-7a-5p dans SEQ ID N° : 31, TGATGTAGTAGGTTGTATAG
iso-hsa-let-7b-5p dans SEQ ID N° : 32, TGAGGTAGTAGGTTGTGTGGTTTA
iso-hsa-miR-100-5p dans SEQ ID N° : 33, AACCCGTAGATCCGAACTTGT
iii. Détermination du nombre de lectures pour 14 miARN biomarqueurs mentionnés dans l'étape ii);
dans laquelle le résultat de l'évaluation est considéré comme positif si les valeurs seuils ≥ 1 pour le nombre de lectures de 1;
et
sachant que si le résultat de l'évaluation des deux analyses est positif, alors la prédiction de la réussite du processus de fécondation *in vitro* l'est également.

2. Méthode selon la revendication 1, **caractérisée par le fait que** l'évaluation de l'identification des miARN canoniques et des iso-miARN choisis en tant que les biomarqueurs pronostiques, détectés dans le plasma obtenu à partir d'un échantillon de sang périphérique de la femme prélevé au jour de la réalisation du processus de fécondation *in vitro,* avec une précision de prédiction de 97 % établie par la méthode ASC, comprend les étapes suivantes:
a) Prélèvement d'un échantillon de plasma préparé à partir de sang périphérique pris d'une femme;
b) Isolement de l'ensemble des miARN, suivi d'une analyse des séquences et d'une analyse des données pour les 10 miARN et/ou iso-miARN choisis comme biomarqueurs, classés en fonction de leur importance pour le résultat du processus de fécondation *in vitro:*
iso-hsa-miR-126-3p dans SEQ ID N° : 1, CGTACCGTGAGAAATAATGCGT
iso-hsa-let-7i-5p dans SEQ ID N° : 2, TGTGGTAGTAGTTTGTGCTGTA
iso-hsa-miR-342-3p dans SEQ ID N° : 3, TCTCACACAGAAATCGGACCCGTCT
iso-hsa-let-7b-5p dans SEQ ID N° : 4, TGAGGTAGTAGGTTGTGTGTTA
iso-hsa-miR-132-3p dans SEQ ID N° : 5, TAACAGCCTACAGCCATGGTCGT
iso-hsa-miR-23b-3p dans SEQ ID N° : 6, GCACATTGCCAGGGATTACCACT
iso-hsa-miR-125a-5p dans SEQ ID N° : 7, TCCCTGAGACCTTTTAACCTGT
iso-hsa-miR-584-5p dans SEQ ID N° : 8, TATGGTTTGCCTGGGACTG
iso-hsa-miR-127-3p dans SEQ ID N° : 9, TCGGATCAGTCTGAGCTTGGCTTTT
iso-hsa-miR-151a-3p dans SEQ ID N° : 11, TACTAGACTAAAGCTCCTTGAGGT
c) Détermination du nombre de lectures pour 10 miARN biomarqueurs mentionnés dans l'étape b).

3. Méthode selon la revendication 1, **caractérisée par le fait que** l'évaluation de l'identification des miARN canoniques et des iso-miARN choisis en tant que les biomarqueurs pronostiques, détectés dans le plasma obtenu à partir d'un échantillon de sang périphérique de la femme prélevé au jour de la réalisation du processus de fécondation *in vitro,* avec une sensibilité de 100 % et une spécificité de 100 %, comprend les étapes suivantes:
a) Prélèvement d'un échantillon de plasma préparé à partir de sang périphérique pris d'une femme;
b) Isolement de l'ensemble des miARN, suivi d'une analyse des séquences et d'une analyse des données pour les 19 miARN choisis, classés en fonction de leur importance pour le résultat du processus de fécondation *in vitro :*
iso-hsa-miR-126-3p dans SEQ ID N° : 1, CGTACCGTGAGAAATAATGCGT
iso-hsa-let-7i-5p dans SEQ ID N° : 2, TGTGGTAGTAGTTTGTGCTGTA
iso-hsa-miR-342-3p dans SEQ ID N° : 3, TCTCACACAGAAATCGGACCCGTCT
iso-hsa-let-7b-5p dans SEQ ID N° : 4, TGAGGTAGTAGGTTGTGTGTTA
iso-hsa-miR-132-3p dans SEQ ID N° : 5, TAACAGCCTACAGCCATGGTCGT
iso-hsa-miR-23b-3p dans SEQ ID N° : 6, GCACATTGCCAGGGATTACCACT
iso-hsa-miR-125a-5p dans SEQ ID N° : 7, TCCCTGAGACCTTTTAACCTGT
iso-hsa-miR-584-5p dans SEQ ID N° : 8, TATGGTTTGCCTGGGACTG
iso-hsa-miR-127-3p dans SEQ ID N° : 9, TCGGATCAGTCTGAGCTTGGCTTTT
iso-hsa-miR-101-3p dans SEQ ID N° : 10, TACACTACTGTGATAACTGA
iso-hsa-miR-151a-3p dans SEQ ID N° : 11, TACTAGACTAAAGCTCCTTGAGGT
iso-hsa-miR-92a-3p dans SEQ ID N° : 12, GATTGCACTTGTCCCGGCCTGAA
iso-hsa-let-7b-5p dans SEQ ID N° : 13, TGAGGTAGTATGTTGTGTGG
iso-hsa-miR-589-5p dans SEQ ID N° : 14, TGAGAACCACGTCCGCTCTGAGC
iso-hsa-miR-652-3p dans SEQ ID N° : 15, GATGGCGCCACTAGGGTTGTGA
iso-hsa-miR-1307-3p dans SEQ ID N° : 16, ACTCGGCGTAGCGTCGGTCGTGT
iso-hsa-miR-382-5p dans SEQ ID N° : 17, GAAGTTGTTCGCGGTGGATTC
iso-hsa-let-7b-5p dans SEQ ID N° : 18, TGAGGTAGTAAGTTGTGTGGTAT
iso-hsa-let-7i-5p dans SEQ ID N° : 19, TGAGTTAGTAGTTTGTGCTGTTTT
c) Détermination du nombre de lectures pour les 19 miARN et/ou iso-miARN biomarqueurs mentionnés dans l'étape b).

4. Méthode selon la revendication 1, **caractérisée par le fait que** l'évaluation de l'identification des miARN canoniques et des iso-miARN choisis en tant que les biomarqueurs pronostiques, détectés dans l'échantillon du milieu utilisé pour la culture de blastocyste, avec une sensibilité de 100 % et une spécificité de 80 %, comprend les étapes suivantes:
i. Prélèvement d'un échantillon du milieu utilisé pour la culture de blastocyste ;
ii. Isolement de l'ensemble des miARN et/ou des iso-miARN servant de biomarqueurs, suivi d'une analyse des séquences et d'une analyse des données pour les 17 miARN et/ou iso-miARN choisis comme biomarqueurs, classés en fonction de leur importance pour le résultat du processus de fécondation *in vitro:*
iso-hsa-let-7b-5p dans SEQ ID N° : 20, TGAGGTGGTAGGTTGTGTGGT
iso-hsa-miR-423-5p dans SEQ ID N° : 21, TGAGGGGCAGAGAGCGAGACTTA
iso-hsa-miR-21-5p dans SEQ ID N° : 22, TAGCTTACCAGACTGATGTTGAC
iso-hsa-miR-486-5p dans SEQ ID N° : 23, TCCTGTACTGAGCTGCCCAGAGA
iso-hsa-miR-486-5p dans SEQ ID N° : 24, TCCTGTACTGAGCTGCCTCGAG
iso-hsa-miR-25-3p dans SEQ ID N° : 25, CATTGCAATTGTCTCGGTCTGA
iso-hsa-miR-3613-5p dans SEQ ID N° : 26, TGTTGTACTTTTTTTTTTGTT
iso-hsa-miR-142-3p dans SEQ ID N° : 27, TGTAGTGTTTCCTACCTTATGGA
iso-hsa-miR-664a-5p dans SEQ ID N° : 28, ACTGGCTAGGGAAAATGATTGGA
iso-hsa-miR-151a-3p dans SEQ ID N° : 29, CAAGACTGAAGCTCCTTGAGG
iso-hsa-miR-92a-3p dans SEQ ID N° : 30, TATCGCACTTGTCCCGGCCTGT
iso-hsa-let-7a-5p dans SEQ ID N° : 31, TGATGTAGTAGGTTGTATAG
iso-hsa-let-7b-5p dans SEQ ID N° : 32, TGAGGTAGTAGGTTGTGTGGTTTA
iso-hsa-miR-100-5p dans SEQ ID N° : 33, AACCCGTAGATCCGAACTTGT
iso-hsa-miR-191-5p dans SEQ ID N° : 34, CAATGGAATCCCAAAAGCAGCTG
iso-hsa-let-7a-5p dans SEQ ID N° : 35, TGAGTTAGTAGGTTGTATAG
iso-hsa-let-7d 5p dans SEQ ID N° : 36, AGAGGTAGTAGGTTGCGTAGTT
iii. Détermination du nombre de lectures pour les 17 miARN et/ou iso-miARN biomarqueurs mentionnés dans l'étape ii).

5. Méthode selon les revendications 2 ou 3, **caractérisée par le fait que** la valeur seuil de 1 pour les miARN canoniques et/ou les iso-miARN choisis en tant que les biomarqueurs pronostiques, détectés dans le plasma obtenu à partir d'un échantillon de sang périphérique de la femme prélevé au jour de la réalisation du processus de fécondation *in vitro,* définit les molécules de miARN ayant une valeur ≥ 1 comme étant les molécules prédisant la réussite du processus de FIV, tandis que celles ayant une valeur < 1 comme étant les molécules prédisant l'échec du processus de FIV, et la valeur seuil de 5 définissant les molécules de miARN ayant une valeur ≥ 5 comme étant les molécules prédisant la réussite du processus de fécondation *in vitro,* tandis que celles ayant une valeur < 5 comme étant les molécules prédisant l'échec du processus de fécondation *in vitro.*

6. Méthode selon les revendications 1 ou 4, **caractérisée par le fait que** la valeur seuil de 1 pour les miARN canoniques et/ou les iso-miARN choisis en tant que les pronostiques, détectés dans l'échantillon du milieu utilisé pour la culture de blastocyste et prélevé au jour 4 à 6 de la culture de l'embryon, définit les miARN ayant une valeur ≥ 1 comme étant les molécules prédisant un embryon avec une haute capacité de développement (c'est-à-dire un embryon de haute qualité) et donc prédisant la réussite du processus de fécondation *in vitro,* tandis que les valeurs < 1 prédisent un embryon avec une faible capacité de développement (c'est-à-dire un embryon de faible qualité) et donc prédisent l'échec du processus de fécondation *in vitro.*
